# EUROPEAN PATENT APPLICATION

(11) **EP 2 158 848 A1**
(43) Date of publication of application: **03.03.2010**
(21) Application number: 08765465.3
(22) Date of filing: 11.06.2008
(51) Int. Cl.: A61B 17/11

(54) **SPRAY HEAD, APPLICATOR FOR APPLYING BIOLOGICAL TISSUE BONDING AGENT, AND METHOD OF APPLYING BONDING AGENT**

(30) Priority: 15.06.2007 JP 2007159047
(71) Applicant: Juridical Foundation The Chemo-Sero-Therapeutic Research Institute, Kumamoto-shi Kumamoto-ken 860-8568 (JP)
(72) Inventor: IFUKU, Yoshihiro, Kumamoto-shi Kumamoto 860-8568 (JP); FUJIWARA, Keisuke, Kumamoto-shi Kumamoto 860-8568 (JP); KAIHARA, Noriyasu, Kumamoto-shi Kumamoto 860-8568 (JP); MIYAZAKI, Yasunari, Kumamoto-shi Kumamoto 860-8568 (JP)
(74) Representative: Vossius & Partner
(86) International application number: PCT/JP2008/060686
(87) International publication number: WO 2008/153059

(57) **Abstract**

A spray head 30 for use with a biological tissue adhesive applicator 1 for spraying and mixing solutions accommodated in a pair of syringes 2(2a, 2b) respectively to obtain a biological tissue adhesive to be applied to surgical sites has a first ejection port 42a for ejecting the solution accommodated in one syringe 2a in a predetermined ejecting direction, and a second ejection port 42b for ejecting the solution accommodated in the other syringe 2b in a direction which is the same or substantially the same as said ejecting direction, the second ejection port 42b being disposed forward of the first ejection port 42a. According to the invention, when applying the biological tissue adhesive made by simultaneously spraying and mixing the first and second solutions accommodated in the first and second syringes respectively, no plugging occurs at the ejection ports for ejecting first and second solutions, ensuring intermittent applications of the biological tissue adhesive.

## Description

### FIELD OF THE INVENTION

The present invention relates to an apparatus for applying a biological tissue adhesive to surgical sites, the adhesive being obtained by simultaneously spraying and mixing a first solution containing a first component such as animal protein and a second solution containing a second component for accelerating coagulation reaction, of the first component. Alto, the present invention relates to a spray head for use with the apparatus. Further, the present invention relates to a method for applying a adhesive such as biological tissue adhesive.

### BACKGROUND OF THE INVENTION

Conventionally, the biological tissue adhesive containing animal protein in its major proportion is produced in clinical practices by mixing one solution (first solution) containing fibrinogen with the other solution (second solution) containing thrombin.

In order for the biological tissue adhesive to be readily and effectively used in clinical practices, a variety of techniques in each of which the first solution accommodated in the first syringe and the second solution accommodated in the second syringe are simultaneously ejected therefrom respectively and mixed with each other to apply the mixture on the surgical sites are disclosed in, among others, JP 64-25843 A, JP 64-40040 A, JUM 3-181979 B, JP 2741323 B, JP 2837099 B, JP 8-281150 A, and JP 11-76245 A. The applicant developed a biological tissue adhesive applicator for spraying the first and second solutions and thereby mixing the sprayed solutions with each other. This applicator is disclosed in JP 2555549 B and has been provided to the practical clinical sites. Through the developments and services of the applicator, the biological tissue adhesive has been widely used as adhesive or hemostatic agent in various medical areas.

In such applicators, the first and second solutions are accommodated in respective syringes independently to prevent them from making contacts or mixing with each other, which eventually prevents any generation of coagulation which may otherwise be caused by the mixing of two solutions or the resultant plugging of the ejections ports of the solutions. Practically, however, the intermittent ejections of the solutions may cause coagulation in the vicinities of the ejection ports (in particular, the ejection port for the first solution containing fibrinogen), by the mixing of the solutions remaining adjacent the ports. It can be understood that the coagulation reaction is caused by air flow formed around the ports at the ejection of the solutions, dripping of the solutions caused after the ejections, and/or bouncing of the solutions from the surgical sites. The coagulation so caused may plug the ejection port or ports to prevent subsequent ejections of the solutions.

Accordingly, the present invention is to provide an improvement to the technology in which the first solution accommodated in the first syringe and the second solution accommodated in the second syringe are simultaneously sprayed and mixed with each other, wherein the improvement prevents the ejection port or ports from being plugged by the mixture of the solutions and allows the intermittent applications of the biological tissue adhesive.

### SUMMARY OF THE INVENTION

To this end, a spray head according to the invention is for use with a biological tissue adhesive applicator for spraying and mixing solutions accommodated in a pair of syringes respectively to obtain a biological tissue adhesive to be applied to surgical sites and comprises a first ejection port for ejecting the solution accommodated in one syringe in a predetermined ejecting direction; and a second ejection port for ejecting the solution accommodated in the other syringe in a direction which is the same or substantially the same as said ejecting direction, the second ejection port being disposed forward of said first ejection port.

A method for spraying and mixing a first solution and a second solution accelerating a coagulation reaction of the first solution, according to the invention comprises ejecting the first solution from a first ejection port, and ejecting said second solution from a second solution port, the second ejection port being disposed forward of the first ejection port with respect to a direction in which the first solution is ejected from the first ejection port.

With the invention, the arrangement that the second ejection pert is disposed forward of the first ejection port with respect to the ejecting direction prevents the first and second solutions from being mixed with each other and, as a result, a plugging of the fist ejection port by any coagulation which would otherwise be caused by the mixing of the solutions when the first and second solutions are ejected from respective ejection ports. Also, the plugging of the second port is prevented by the fact that one solution containing a first component (fibrinogen solution, for example) is used for the first solution and the other solution containing a second component capable of accelerating a coagulation reaction of the first component (thrombin solution, for example) is used for the second solution in order to prohibit an active coagulation reaction even if a small amount of first solution were mixed in the second solution. Therefore, according to the invention, no plugging occurs at the first or second ejection port, which ensures the intermittent applications of the adhesive.

### BRIEF DESCRIPTION OF THE DRAWINGS

Fig. 1 is an exploded perspective view of a biological tissue adhesive applicator according to a first embodiment;

Fig. 2 is a plan view of the assembled applicator shown in Fig.1;

Fig. 3 is a side elevation of the assembled applicator shown in Fig. 1;

Fig. 4 is a horizontal cross section of a spray head according to a first embodiment of Fig.1;

Fig. 5 is a vertical cross section of the spray head shown in Fig. 4;

Fig. 6 is an exploded perspective view of a biological tissue adhesive applicator according to a second embodiment;

Fig. 7 is a plan view of the assembled applicator shown in Fig. 6;

Fig. 8 is a vertical cross section of a spray head according to the second embodiment;

Fig. 9 is a horizontal cross section of the spray head shown in Fig. 8;

Fig. 10 is an exploded perspective view of a conventional applicator used in experiments 3 and 5;

Fig. 11 is an exploded perspective view of an applicator according to the invention, which is used in experiments 2, 3, and 5; and

Fig. 12 is an exploded perspective view of an applicator according to the invention, which is used in experiment 4.

### DETAILED DESCRIPTION OF THE PREFERRED EMBODIMENTS

Hereinafter, preferred embodiments of the present invention will be described with reference to the accompanying drawings. In the following description, terminologies referring to specific directions (for example, "up", "down", "left", "right", and others including these terminologies) will be used. It should be noted that these terms are used for the purpose of facilitating the understanding of the invention taken in conjunction with the drawings and meanings of these terms should not be construed as restricting the present invention.

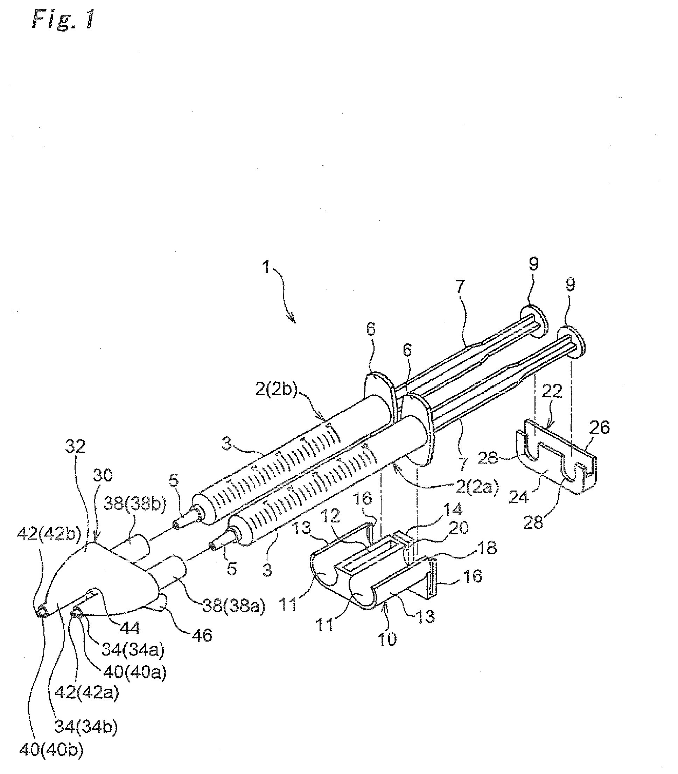

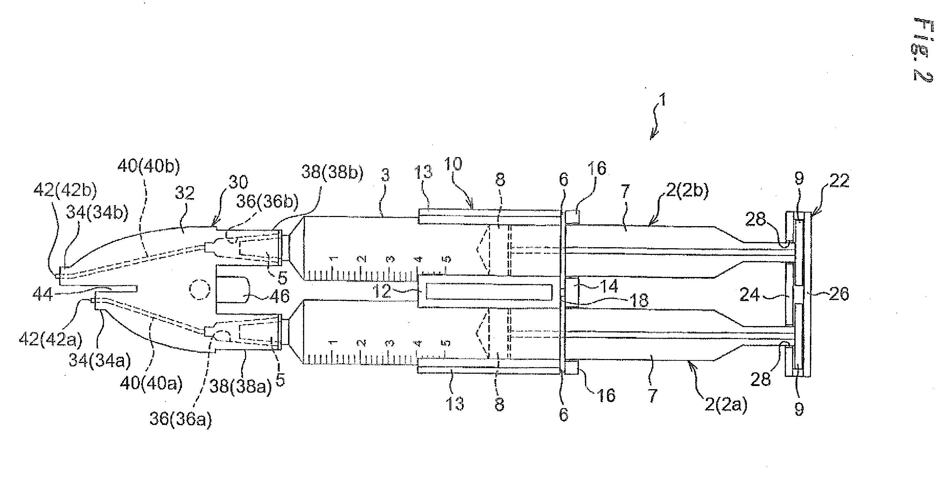

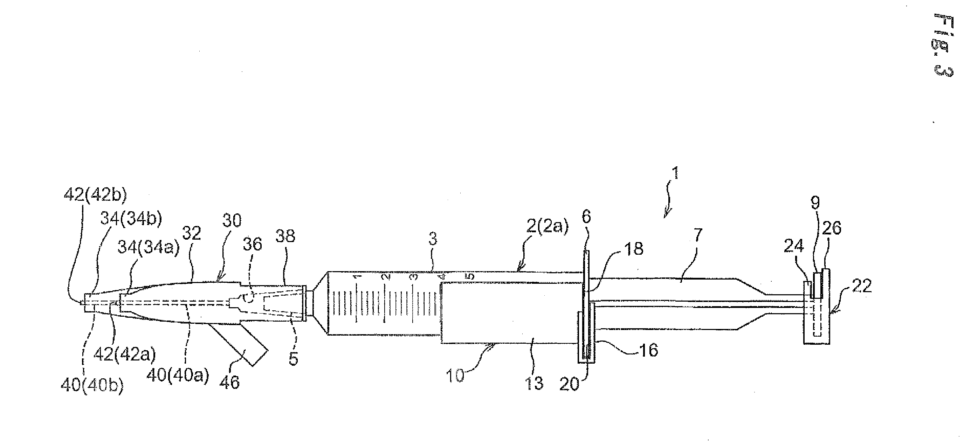

Referring now to Figs. 1-5, a first embodiment of the present invention will be described. As shown in Figs. 1-3, a biological tissue adhesive applicator, generally indicated by reference numeral 1, according to the first embodiment of the invention has a first syringe 2a for accommodating fibrinogen solution (first solution) including fibrinogen (first component) and a second syringe 2b for accommodating thrombin solution (second solution) including thrombin (second component) capable of accelerating the coagulation reaction of fibrinogen, and a spray head 30 for spraying the fibrinogen and thrombin solutions supplied from the syringes 2 (2a, 2b), respectively.

Each syringe 2 has a barrel 3 and a plunger 7. The barrel 3 has a cylindrical body 4, a nozzle 5 at one end of the cylindrical body 4, and flanges 6 at the other end of the cylindrical body 4 and extending radially outwardly, formed integrally therewith. The plunger 7 has a gasket made of rubber for example and mounted at one end thereof, having a diameter slightly larger than the inner diameter of the cylindrical body, and a thumb yoke 9 integrally made therewith. The one end of the plunger 7 carrying the gasket 8 is inserted in the interior of the cylindrical body 4 of the barrel 3 so that the gasket 8 makes a sealing contact with the inner peripheral surface of the cylindrical body 4- The plungers 7 may have different colors so that users can readily identify the solutions accommodated in respective syringes 2. For example, one plunger for the syringe 2a accommodating the fibrinogen solution is blue-colored and the other plunger for the syringe 2b accommodating the thrombin solution is red-colored.

The two barrels 3 are held by a syringe holding member or holder 10. The holder 10 is integrally formed of synthetic resin such as polyethylene, polypropylene, or acrylonitrile-butadiene-styrene copolymer and has two grooves 11 extending in parallel to each other. Each groove 11 has a C-shaped inner surface which substantially corresponds to the outer configuration of the cylindrical body 4. A transverse width of the top opening of the groove 11 is smaller than the outer diameter of the cylindrical body 4, which allows the holder 10 to securely hold the barrel 3 in the groove 11 when the barrel. 3 is fitted in the groove 11.

The holder 10 has a stop 14 opposing a rear end of the connecting portion 12 positioned between the grooves 11 while leaving a certain gap therebetween and stops 16 opposing respective rear ends of the outward walls 13 defining in part the grooves 11. The stops 14 and 16 cooperate with the rear end surfaces 18 opposing thereto to define a pair of flange receiving grooves 20 so that, when the barrels 3 are fitted in respective grooves 11, the flanges 6 of the barrels 3 are also fitted in respective grooves 20.

The plungers 7 inserted in respective barrels 3 are simultaneously actuated by the use of an actuator 22. The actuator 22, which is preferably made of the same material as that of holder 10, has a front wall 24 and a rear wall 26 opposing the front wall 24 while leaving a certain gap therebetween. Preferably, the gap between the front and rear walls 24 and 26 is determined to be substantially the same as the thickness of the thumb yoke 9 of the plunger 7. The front wall 24 has two upwardly-opened cutouts 28 in the form "U", for example. A distance between the centers of the cutouts 28 is determined to be the same as that of the grooves 11.

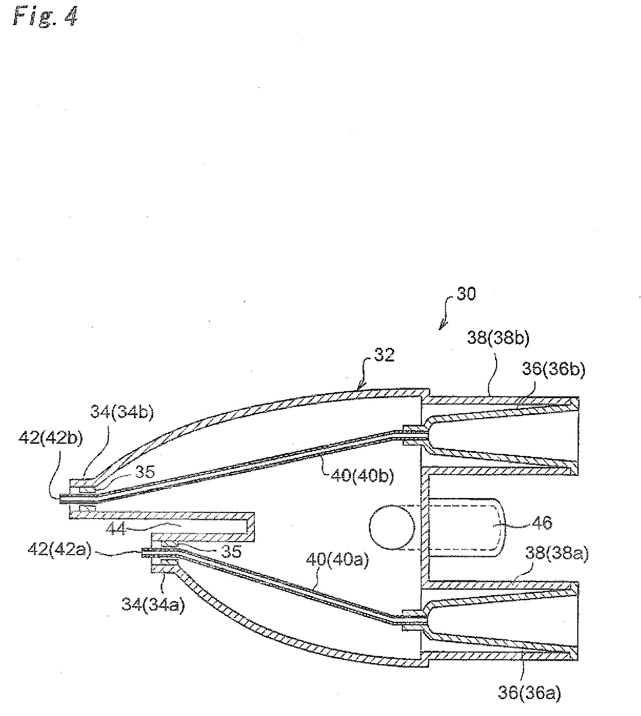

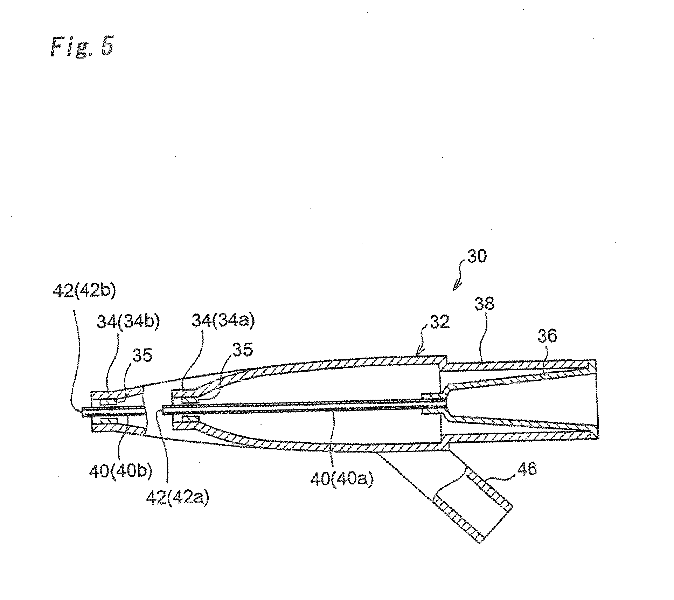

As shown in Figs. 4 and 5, the spray head 30 has a flattened hollow housing 32. The housing 32 has a pair of first and second gas ejection nozzles 34 (34a, 34b) each defined by the cylindrical walls at the front end of the housing 32. The interiors of the gas ejection nozzles 34 are connected with the interior of the housing 32. The gas ejection nozzles 34a and 34b are so disposed that the longitudinal axes of the nozzles orient in the same or substantially the same ejecting direction.

The second gas ejection nozzle 34b is disposed forward of the first gas ejection nozzle 34a with respect to the ejecting direction. The outer peripheries of the gas ejection nozzles 34a, 34b are spaced apart from each other to define a recess 44 extending in the rearward direction. The spaced-apart arrangement of the nozzles 34a, 34b prevents the solution possibly staying at the distal end of one gas ejection nozzle 34 from moving along the outer peripheral surface of its gas ejection nozzle to meet the other solution possibly staying at the distal end of the other gas ejection nozzle 34. It is not necessary for the peripheral surfaces of gas ejection nozzles 34a, 34b are transversely spaced apart from each other. Alternatively, at least the distal end peripheral portion of the first gas ejection nozzle 34a may be spaced apart from the peripheral surface of the second gas ejection nozzle 34b.

Each of the gas ejection nozzles 34 has a plurality of ribs 35 formed therewith and extending radially inwardly to define a space surrounded by the ribs 35 in which a solution tube 40 is inserted.

The housing 32 has a pair of syringe connectors 38 (38a, 38b) formed therewith at the rear end thereof. The syringe connectors 38a, 38b receive first and second adaptors 36a, 36b engaged therein respectively for receiving the nozzles 5 of the first and second syringes 2a, 2b fitted therein. Preferably, the syringe connectors 38 are colored in respective colors which are different from each other but the same as those of the corresponding plungers 7 of the syringes 2, which allows users to identify the syringes 2 to be connected to respective syringe connectors 38 at the assembling of the syringes 2 to the spray head 30. For example, the syringe connector 38a is blue-colored corresponding to the first syringe 2a and the syringe connector 38b is red-colored corresponding to the second syringe 2b. The adaptors 36 (36a, 36b) are tapered forwardly so that the nozzles 5 of the syringes 2 are well fitted therein. Also, the adaptors 36 have distal end openings defined therewith,

First and second solution tubes 40 (40a, 40b) are disposed within the interior of the housing 32. One end of the solution tube 40a. is connected to the distal end opening of the first adaptor 36a and the other end thereof is inserted in the first gas ejection nozzle 34a- one end of the second solution tube 40b is connected to the distal end opening of the second adaptor 36b and the other end thereof is inserted in the second gas ejection nozzle 34b. The other ends of the solution tubes 40 are projected a certain distance from the gas ejection nozzles 34, which results in that the distal end of the first solution tube 40a defines a first ejection nozzle 42a for ejecting the fibrinogen solution and the distal end of the second solution tube 40b defines a second ejection nozzle 42b for ejecting the thrombin solution. Preferably, the projected length of the tubes 40 are 0.1-10mm, for example, which ensures that the solutions being ejected from the tubes 40 are uniformly sprayed with an aid of sterile gas ejected from the gas ejection nozzles 34.

A distance between the longitudinal central axes of the ejection nozzles 42a, 42b is set preferably in the range of 2-10mm, or more preferably in the range of 3-6mm. It should be noted that those ranges are not restrictive to the present invention and may be larger or smaller if the directions along which the solutions are ejected are nonparallel to each other due to, for example, inclinations of the solution tubes 42a, 42b. The ejection nozzles 42a, 42b take different positions with respect to the ejecting direction so that the second ejection nozzle 42b is disposed forward of the first ejection nozzle 42a with respect to the direction. The distance between the ejection nozzles 42a, 42b with respect to the ejecting direction is determined by taking account of the transverse distance of the nozzles and spraying angle of the solution from the rearward first ejection nozzle 42a so that the ejected solution from the first ejection nozzle 42a does not make contact with the forward second ejection nozzle 42b. The distance is set preferably in the range of 5-16 mm, or more preferably in the range of 6-8mm, which are not restrictive to the invention.

The spray head 30 also has a gas supply tube extending downwardly and rearwardly from the bottom wall thereof. Although the gas supply tube 46 is integrally formed with the housing 32, it may be manufactured independently and connected to the bottom wall of the housing 32. Although not shown, during the operation of the applicator 1, the gas supply tube 46 is connected to a gas supply source through a filter so that a gas is sterilized by the filter and then supplied through the gas supply tube 46 into the interior of the housing 32. A filter unit commercially available from Millipore under the trade name Millex FG is preferably used for the filter.

Before using the applicator 1 so constructed, a certain amount of fibrinogen solution is accommodated in the first syringe 2a and the same amount of, for example, thrombin solution is accommodated in the second syringe 2b.

Then, the barrels 3 of the syringes 2 are fi tted in respective grooves 11 of the holder 10 and the flanges 6 of the barrels 3 are also fitted in the flange receiving grooves 20. The transverse widths of the top openings of the grooves 11 are larger than the outer diameter of the barrels so that the barrels 3 are well retained by the holder 3 without dropping therefrom.

Also, the thumb yokes 9 of the plungers 7 are fitted between the front and rear walls 24 and 26 of the actuator 22 and the rear end portions of the plungers 7 adjacent the thumb yokes 9 are fitted in the cutouts 28 of the front wall 24. Since the gap between the front and rear walls 24 and 26 is substantially the same as the thickness of the thumb yokes 9, the actuator 22 is well retained on the plungers 7.

Further, the nozzle 5 of the first syringe 2a accommodating the fibrinogen solution is fitted in the first adaptor 36a of the spray head 30 and the nozzle 5 of the second syringe 2b accommodating the thrombin solution is fitted on the second adaptor 36b of the spray head 30.

Furthermore, the gas supply tube 46 of the spray head so is connected through the filter to a tube extending from the gas supply not shown.

When using the applicator 1 so assembled, the sterile gas is supplied into the interior of the housing 32 of the spray head 30 through the gas supply tube 46 from the gas supply and then ejected from the gas ejection nozzles 34.

An operator places his or her index and/or mid finger on the holder 10 and/or flange 6 and thumb finger on the rear surface of the actuator 22 and forces the plungers 7 into respective barrels 3, which causes the solutions to be ejected from the solution tubes 40. The solutions ejected from respective tubes 40 are sprayed with an aid the sterile gas ejected therearound and then the sprayed solutions are mixed with each other and applied on the surgical sites. The tubes 40 inserted in respective gas ejection nozzles 34 are retained in a stable manner by the ribs 35 disposed therearound, so that the solutions from the nozzles 42 (42a, 42b) of the tubes 40 are sprayed in the predetermined directions, which ensures the mixtures to be applied to the target sites.

In particular, the second ejection nozzle 42b is placed forward of the first ejection nozzle 42a with respect to the ejecting direction. This prevents the thrombin solution from the second ejection nozzle 42b from making contacts with the fibrinogen solution in the vicinity of the first ejection nozzle 42a to generate any coagulation which may plug the first ejection nozzle 42a.

As described above, the longitudinal distance between the first and second ejection nozzles 42 is so determined as to prevent the fibrinogen solution ejected from the first ejection nozzle 42a from making contacts with the second ejection nozzle 42b. However, it may occur that, due to the air flow generated at the ejections of the solutions, only a small amount of fibrinogen solution ejected from the first ejection nozzle 42a adheres to the second ejection nozzle 42b to mix with the thrombin solution. In this instance, however, even if mixed in the thrombin solution, this small amount of fibrinogen solution does not drive an active chemical reaction, with the thrombin solution to cause unwanted plugging of the second ejection nozzle 42b.

As described above, according to the spray head 30, no plugging occurs at the first and/or the second ejection nozzle 42a, 42b, which ensures the intermittent application of the biological tissue adhesive.

Also, the larger gap defined between the spaced apart gas ejection nozzles 34a, 34b effectively reduces the possibility that the solution remaining at one ejection nozzle 34 moves along the peripheries of the nozzles to reach the other ejection nozzle 34 and then mixes with the other solution during the time periods between the ejecting operations.

The housing 2 of the spray head 30 may be provided with a forward extension at its front end having a length of 5-50cm, for example. In this modification, the distal end of the extension may be formed with a pair of gas ejection nozzles as described above. This allows the solutions to be sprayed uniformly in the deep portions of living bodies.

Also, a gun-type applicator disclosed in JP 2555549 B, for example, may be combined with the biological tissue adhesive applicator 1.

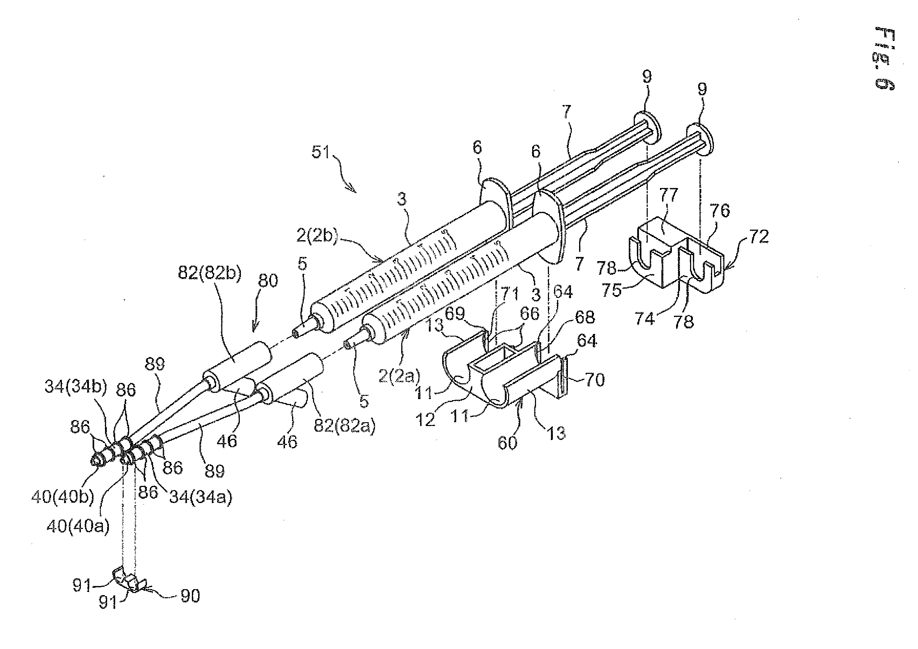

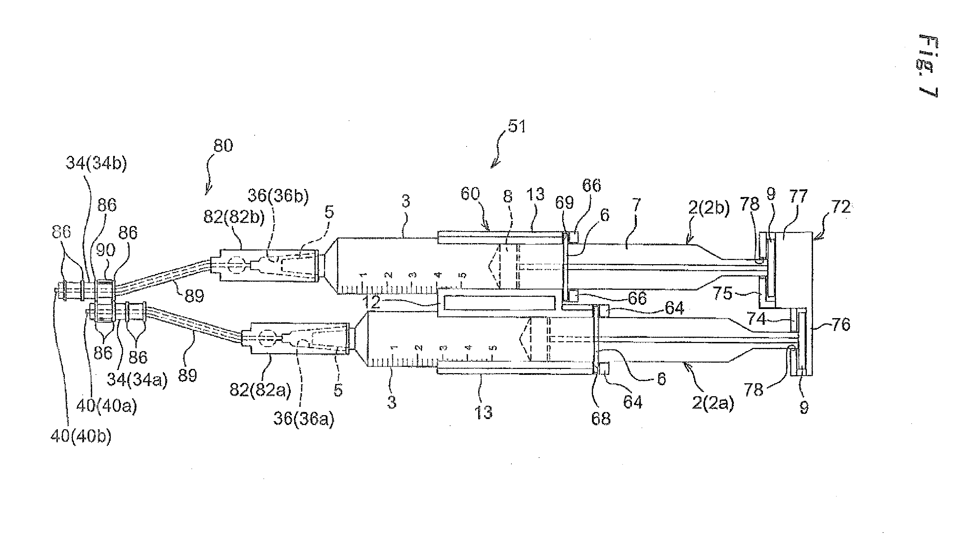

Referring next to Figs. 6-9, a second embodiment of the present invention will be described below. As shown in Figs. 6 and 7, similar to the first embodiment of the invention, the biological tissue adhesive applicator 51 according to the second embodiment of the invention has a first syringe 2a for accommodating fibrinogen solution, a second syringe 2b accommodating thrombin solution, and a spray head 80 for spraying the fibrinogen and thrombin solutions from respective syringes. The detailed structures of the syringes are the same as those of the first embodiment.

Similar to the first embodiment, the applicator 51 has a holder 60 for integrally holding two barrels 3. Also similar to the first embodiment, the holder 60 has two grooves 11 defined therewith. According to the embodiment, the grooves 11 have different longitudinal lengths. Specifically, the rear end portion 68 for one groove 11 is displaced rearward of the rear end portion 69 for the other groove 11. The stops 64, 66 are provided to oppose the rear end portions 68, 69 leaving a gap to form the flange receiving grooves 70, 71 for receiving the flanges 6 of the barrels 3 fitted in the grooves 11, respectively. The arrangement of the flange receiving grooves 70, 71 displaced from each other in the longitudinal direction of the grooves 11 allows the holder 60 to hold the barrels 3 in a displaced manger.

The actuator 72 for simultaneously activating the plungers 7 inserted in respective barrels 3 has two front walls 74, 75 and two rear walls 76, 77. The front walls 74, 75 are displaced from each other in the longitudinal direction corresponding to the displacement of the grooves 70, 71. Likewise, the rear walls 76, 77 are displaced from each other in the longitudinal direction. The front wall 74, 75 have cutouts 78 opened upwardly, in the form of "U". The distance between the centers of the cutouts 78 is the same as that of grooves 11 of the holder 60.

The spray head 80 has a first hollow housing 82a to be attached to the first syringe 2a, a second hollow housing 82b to be attached to the second syringe 2b, a first gas ejection nozzle 34a connected to the first housing 82a through a connection pipe or, and a second gas ejection nozzle 34b connected to the second housing 82b through a connection, pipe 89. In this arrangement, an interior of the first gas ejection nozzle 34a is connected to that of the first housing 82a and an interior of the second gas ejection nozzle 34b is connected to that of the second housing 82b. It should be noted that the gas ejection nozzles 34(34a, 34b) may be directly connected to respective housings 82(82a, 82b) without using connection pipes 89.

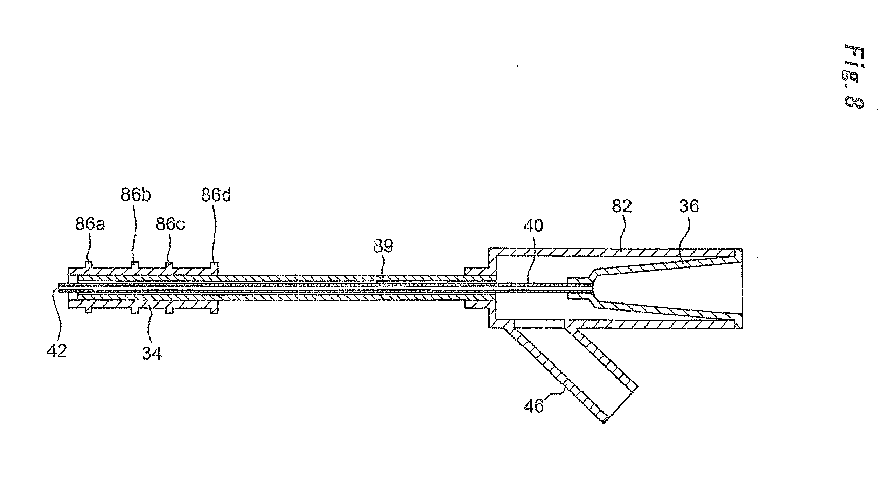

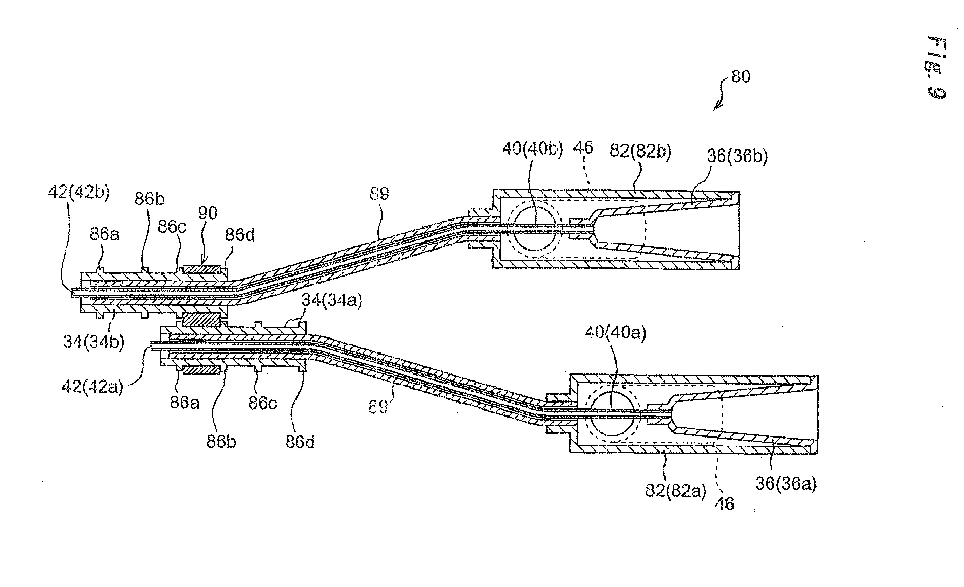

As shown in Figs. 8 and 9, the first housing 82a receives a first adaptor 36a mounted therein and the second housing 82b has a second adaptor 36b mounted therein. Preferably, the housings 82 (82a, 82b) are colored in the same colors as the associated plungers 8, respectively, so that operators can see easily which syringe 2 should be connected tao which housing 82 at the assembling of the syringes 2 to the associated spray heads 80. For example, the first housing 82a is as the first syringe 2a and the second housing 82b is red-colored as the second syringe 2b.

Each of the gas ejection nozzles 34 has a cylindrical wall which bears a plurality of flanges 86 (86a, 86b, 86c, 86d) extending outwardly therefrom. The flanges 86 are disposed at certain intervals in the ejecting direction. For example, the flanges 86a, 86d are so arranged that the distance from the first flange 86a to the second flange 86b is the same as that from the third flange 86c to the fourth flange 86d.

The first and second gas ejection nozzles 34a, 34b are retained by the nozzle holder 90. As shown in Fig. 6, the nozzles holder 90 has a pair of grooves 91 extending in parallel to each other. Each groove 91 has a C-shaped inner surface portion which substantially corresponds to the outer peripheral surface portion of the gas ejection nozzle 34. The transverse width of the top opening of the grooves 91 is smaller than the outer diameter of the gas ejection nozzle 39 so that, when the gas ejection nozzles 34 are fitted in the respective grooves 91, they are securely retained by the nozzle holder 90. The longitudinal axes of the gas ejection nozzles 34a, 34b are directed to the same or substantially the same direction in which the solutions are ejected. The longitudinal length of the grooves 91 is substantially the same as the lengths of the portions of the nozzles existing between the neighboring flanges 86, which ensures to prevent any displacement in the ejecting direction of the gas ejection nozzles 34 fitted if the grooves 91. This meant that, simply by fitting the gas ejection nozzles in the nozzle holder 90, the relative positional relation between the gas ejection nozzles 34 is determined. As shown in Figs. 8 and 9, in this embodiment a portion of the first ejection nozzle 34a between the first and second flanges 86a, 86b is fitted in one groove 91 and a portion of the second ejection nozzle 34b between the third and fourth flanges 86c, 86d is fitted in the other groove 91, which ensures that the gas ejection nozzles 34 are positioned properly to meet the predetermined positional relation thereof in which the second ejection nozzle 34b is disposed forward of the first gas ejection nozzle 34a in the gas ejecting direction.

The connection tubes 89 are connected at one ends thereof to the distal end openings of the housings 82a, 82b and at the other ends thereof to the gas ejection nozzles 34, respectively. A first solution tube 40a is inserted in the connection tube 89 connected to the first housing 82a and a second solution tube 40b is inserted in the connection tube 89 connected to the second housing 82b. Preferably, the connection tubes 89 are made of flexible material.

Similar to the first embodiment one end of each solution tube 40 (40a, 40b) is connected to the distal end opening of the adaptor 36 and the other end thereof is inserted in the gas ejection nozzle 34 and projected a predetermined length from the gas ejection nozzle 34 to terminate at its ejection port 42. The two ejection ports 42 are disposed to meet the positional relationship as described above simply by fitting the gas ejection nozzles 34 in the nozzle holder 90.

Each housing 82 has a gas supply tube 46 as described in the first embodiment. In this embodiment, the gas supply tube 46 is extended from the bottom wall of the housing in the downward and rearward direction. The gas supply tube 46 may be manufactured independently.

When using the applicator 51 so constructed, similar to the first embodiment, the fibrinogen solution is accommodated in the first syringe 2a and the thrombin solution is accommodated in the second syringe 2b, having the same amount as the fibrinogen solution, for example. Also, the barrels 3 of the syringes 2 are fitted in the holder 60. Further, the thumb yokes 9 of the plungers 7 are fitted in the actuator 72.

Next, the nozzles 5 of the syringes 2a, 2b are fitted in the associated adaptors 36a, 36b, respectively, and the predetermined portions of the gas ejection nozzles 34a, 34b are mounted on the nozzle holder 90.

Subsequently, the gas supply tube 46 of the spray head 80 is connected through the filter to a tube extending from the gas supply not shown. This allows that the biological tissue adhesive to be applied as described in the first embodiment.

In the application of the adhesive using the applicator 51, since the second ejection port 42b is disposed forward of the second ejection port 42b with respect to the ejecting direction similar to the first embodiment, no plugging occurs at the first or second ejection port 42a, 42b, which allows the applicator 51 to apply the biological tissue adhesive on and off.

In the second embodiment, other structures and the advantages derived therefrom are the same as those of the first embodiment, and like parts are indicated like reference numerals in Figs. 6-9.

The structure for displacing the positions of the ejection ports 42a, 42b with respect to the ejecting direction is not limited to that in which the syringes 2a, 2b are displaced from each other. For example, the second ejection port 42b is displaced forward of the first ejection port 42a by shortening the length of the first solution tube 40a than the second solution tube 40b and also shortening the length of the connection tube 89 inserted in the first solution tube 40a than the connection tube 89 inserted in the second solution tube 40b. Alternatively, the second ejection port 42b is displaced forward of the first ejection port 42a by sagging the first solution tube 40a and the connection tube 89 inserted in the first solution tube 40a, both made of flexible materials.

Although the flanges 86 are provided on the gas ejection nozzles 34 in the second embodiment, they may be removed therefrom. In this modification, the second ejection port 42b may be placed forward of the first ejection port 42a buy sliding at least one gas ejection nozzle away from the other after the mounting of the gas ejection nozzles 34a, 34b on the nozzle holder 90.

Although the present invention has been fully described with the specific embodiments thereof, it is not limited thereto. For example, although in the previous embodiments the fibrinogen solution is ejected from the first ejection port 42 and the thrombin solution is ejected from the second ejection port 42b, the invention can be applied equally to other applicators in which solutions other than fibrinogen and/or thrombin solution is ejected therefrom.

### EXPERIMENT 1

An experiment was conducted to verify the occurrence of coagulation. In the first test, the thrombin solution was mixed in the fibrinogen solution. In the second test, the fibrinogen solution was mixed in the thrombin solution.

In the fist test, 1 milliliter of thrombin solution was added in the vial accommodating 3 milliliter of fibrinogen solution and, after a lapse of 10 minutes, the mixture was visually observed. In the second test, 1 milliliter of fibrinogen solution was added in the vial accommodating 3 milliliter of thrombin solution and, after at lapse of 10 minutes, the mixture was visually observed.

The results showed that the mixture of the first test was wholly coagulated and the mixture of the second test was partially coagulated and the amount of coagulation associated with the amount of fibrinogen added.

This shows that, by disposing the ejection port of the fibrinogen solution forward of that of thrombin solution, the fibrinogen solution is not mixed with the thrombin solution at adjacent the rearward ejection port of the thrombin solution and therefore no coagulation occurs. On the other hand, there is a possibility that a small amount of thrombin solution is mixed with the fibrinogen solution at adjacent the forward ejection port of the fibrinogen solution and, if so, there occurs coagulations actively to plug the forward ejection nozzle. If the ejection nozzle of the thrombin solution is placed forward of that of the fibrinogen solution, the fibrinogen solution is not mixed with the thrombin solution at adjacent the rearward ejection nozzle of the fibrinogen solution, so that no coagulation occurs. Also, although a small amount of fibrinogen solution is mixed with the thrombin solution at adjacent the forward ejection nozzle of the thrombin solution, no vigorous reaction occurs. In conclusion, it is determined that no plugging occurs if the ejection port of the thrombin solution is placed forward of that of the fibrinogen solution.

### EXPERIMENT 2

A test was conducted by using the spray head in which the ejection port of the thrombin solution was disposed forward of that of the fibrinogen solution to see if the sprayed fibrinogen and thrombin solutions were well mixed with each other.

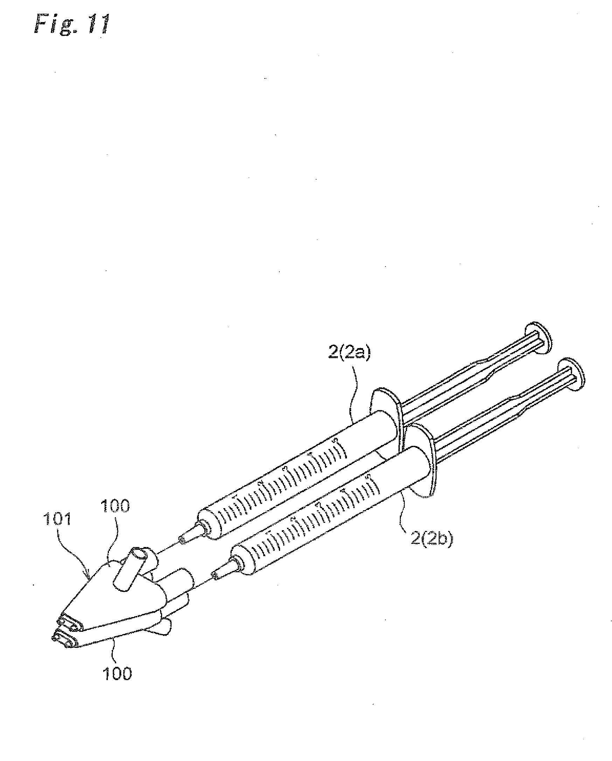

As shown in Fig. 11, a spray head 101 for this test was manufactured by combining two spray heads 100 for use with BOLHEAL spray head commercially available from The Chemo-Sera-Therapeutic Research Institute. In particular, the opposed major surfaces of the spray heads were flattened by fling and then bonded to each other so that the ejection ports were displaced 6-7 millimeters from each other with respect to the ejecting direction.

The syringe accommodating the thrombin solution was connected to the adaptor of the forward spray head and the syringe accommodating the fibrinogen solution was connected to the adaptor of the rearward spray head. In order to prevent the mutual interference of the syringes, the syringes were connected to the diagonally positioned adaptors, so that the centers of the ejection nozzles were spaced 5 millimeters away from each other.

Using the combined spray head to which the syringes were assemble, the solutions were sprayed against a surface positioned about 1-10 centimeters away from the spray head. The solutions were mixed with each other and the adhesive was applied on the surface. This result showed that the sprayed solutions was well mixed with each other to produce the adhesive effectively on the application site by using the arrangement in which the ejection port of the thrombin solution was placed forward of that of the thrombin solution.

### EXPERIMENT 3

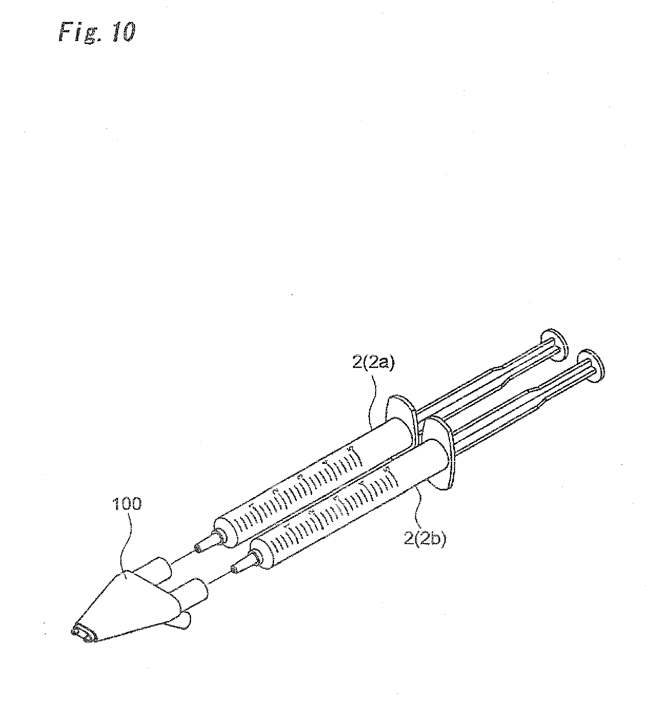

A comparative test was conducted to see the generation of plugging at the ejection ports, by using the combined spray head in test 2 and the conventional spray head shown in Fig. 10.

In the test, the syringes were assembled to the combined and conventional spray heads to spray the solutions every 5 minutes in order to evaluate the performance of the plugging prevention function thereof. The spray heads were mounted on the syringes as described in the experiment 2. The performance of the plugging prevention function were ranked into three levels: Level A in which the fibrinogen and thrombin solutions were well sprayed, Level B in which a slight plugging occurred but nevertheless the solutions were sprayed, and Level C in which the plugging occurred to prevent the spraying, as shown in Table 1.

**TABLE 1**

| No. | Lapsed Time (min. | CONVENTIONAL HEAD | COMBINED HEAD |
|---|---|---|---|
| 1 | 0 | A | A |
| 2 | 5 | A | A |
| 3 | 10 | B | A |
| 4 | 15 | C | A |
| 5 | 20 | B | A |
| 6 | 25 | - | A |

As shown Table 1, in terms of the conventional spray head, the plugging caused at the ejection port for the fibrinogen solution in the fourth spraying (No. 4; Lapsed time was 5 min.), but the solution was sprayed by applying a greater force on the plunger in the fifth spraying. However, all the thrombin solution was completely consumed in the fifth spraying, so that no further spraying (i.e., sixth spraying) could be conducted. In contrast, no plugging occurred and the solutions were well sprayed in the sixth spraying. Also, for the combined head, no fibrinogen solution was observed at adjacent the ejection nozzle of the thrombin solution.

From the experiment 3, it was verified that the arrangement in which the ejection nozzle of the thrombin solution being disposed forward of that of the fibrinogen solution prevents the generation of the plugging at the ejection port and ensures the intermittent applications of the adhesive.

### EXPERIMENT 4

An experiment was conducted to evaluate the plugging prevention performance in another condition different from that of experiment 3, for the combined spray head.

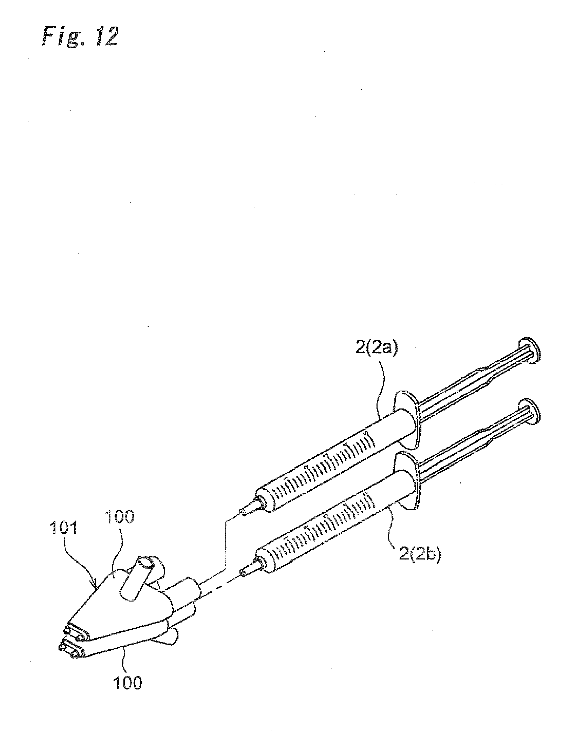

Specifically, in the experiment 4, as shown in Fig. 12, employed were syringes with reduced diameters. The syringes were connected to upper and lower adjacent adaptors, respectively, in which the distance between the centers of the ejection ports was 4 millimeters. The spraying was performed every 30 minutes and the spraying performance was evaluated as in the experiment 3.

The result is indicated in Table 2.

**TABLE 2**

| No. | Lapsed Time (min. | COMBINED HEAD |
|---|---|---|
| | 0 | A |
| 2 | 30 | A |
| 3 | 60 | A |

As shown in table 2, no plugging occurred in each spraying and the solutions were well sprayed. Also, it was verified from the result of experiment 4 that no plugging occurred even when the distance between the centers of the ejection ports was reduced to 4 millimeters and the ejection intervals were increased to 30 minutes.

### EXPERIMENT 5

Another experiment was conducted to evaluate and compare the bonding performances for the combined and conventional spray heads.

The syringes were connected to the combined spray head as in experiment 2. The biological tissue adhesive was applied to artificial blood vessels made of PTFE with a number of small apertures formed by penetrating 5-0 needle. The vessels were closed at the portions having the apertures by applying the biological tissue adhesive. The bonding performance was evaluated by measuring the withstanding pressure of the vessels using a pressure gage commercially available from NIDEC COPAL Corporation under the trade name PG-208-102VH-S.

The result showed that the withstanding pressure obtained by using the conventional spray head was 202.5±75 mmHg and that obtained by using the combined spray head was 247.25±75.25 mmHg. This showed that the substantially the same withstanding pressure was obtained in the conventional and the combined spray head.

Also, it was verified from the experiment 5 that substantially the same bonding performance was obtained when the ejection port of the thrombin solution be disposed forward of that of that of the fibrinogen solution.

## Claims

1. A spray head for use with a biological tissue adhesive applicator for spraying and mixing solutions accommodated in a pair of syringes respectively to obtain a biological tissue adhesive to be applied to surgical sites, comprising:
a first ejection port for ejecting said solution accommodated in one syringe in a predetermined ejecting direction; and
a second ejection port for ejecting said solution accommodated in the other syringe in a direction which is the same or substantially the same as said ejecting direction, said second ejection port being disposed forward of said first ejection port.

2. The spray head of claim 1, further comprising
a pair of gas ejection nozzles having respective central axes oriented in a direction which is the same or substantially the same as said ejecting direction;
a gas supply tube for supplying gas to said gas ejection nozzles;
a first solution tube disposed in said one gas ejection nozzle and defining a solution passage for said solution supplied from said one syringe, said first solution tube terminating at said first ejection port; and a second solution tube disposed in said the other gas ejection nozzle and defining a solution passage for said solution supplied from said the other syringe, said second solution tube terminating at said second ejection port.

3. The spray head of claim 2, further comprising
a hollow housing having a cavity defined therein, said cavity being connected to interiors of said pair of gas ejection nozzles to allow said gas from said gas supply tube to be supplied into said interiors of said gas ejection nozzles.

4. The spray head of claim 2, further comprising
a pair of said gas supply tubes;
a first housing having an interior connected to said interior of said one gas ejection nozzle for supplying said gas from one of said gas supply tubes to said interior of said one gas ejection nozzle; and
a second housing having an interior connected to said interior of said one gas ejection nozzle for supplying said gas from the other of said gas supply tubes to said interior of said one gas ejection nozzle.

5. The spray head in any one of claims 2-4, wherein
each of said gas ejection nozzles is defined by a cylindrical wall extending in a direction which is the same or substantially the same as said ejecting direction; and
at least of a peripheral surface of a distal end of said gas ejection nozzle in which said first solution tube is inserted is spaced apart from that of said gas ejection nozzle in which said second solution tube is inserted.

6. A biological tissue adhesive applicator for spraying and mixing a first solution and a second solution accelerating a coagulation reaction of the first solution to obtain a biological tissue adhesive to be applied to surgical sites, comprising:
a first syringe for accommodating said first solution;
a second syringe for accommodating said second solution; and
a spray head for spraying said first and second solutions supplied from said first and second syringes, respectively,
said spray head having
a first ejection port for ejecting said first solution in a predetermined ejecting direction, and;
a second ejection port for ejecting said second solution in a direction which is the same or substantially the same as said ejecting direction, said second ejection port being disposed forward of said first ejection port with respect to said ejecting direction.

7. The biological tissue adhesive applicator of claim 6, wherein the spray head further comprises
a pair of gas ejection nozzles having respective central axes oriented in a direction which is the same or substantially the same as said ejecting direction;
a gas supply tube for supplying gas to said gas ejection nozzles;
a first solution tube disposed in one of said gas ejection nozzles, said first solution tube terminating at said first ejection port and defining a solution passage for said solution supplied from one of said syringes;
a second solution tube disposed in one of said ejection nozzles, said second solution tube terminating at said second ejection port and defining a solution passage for said solution supplied from the other of said syringes;

8. The biological tissue adhesive applicator of claim 7, wherein the spray head further comprises
a hollow housing having an interior connected to interiors of said gas ejection nozzles, said interior of said hollow housing being connected to said gas supply tube to allow said gas to be supplied from said gas supply tube into said interior of said hollow housing.

9. The biological tissue adhesive applicator of claim 7, wherein the spray head further comprises
a pair of said gas supply tubes;
a first housing having an interior connected to said interior of said one gas ejection nozzle for supplying said gas from one of said gas supply tubes to said interior of said one gas ejection nozzle; and
a second housing having an interior connected to said interior of said one gas ejection nozzle for supplying said gas from the other of said gas supply tubes to said interior of said one gas ejection nozzle

10. The biological tissue adhesive applicator in any one of claims 7-9, wherein
each of said gas ejection nozzles is defined by a cylindrical wall extending in a direction which is the same or substantially the same as said ejecting direction;
at least of a peripheral surface of a distal end of said gas ejection nozzle in which said first solution tube is inserted is spaced apart from that of said gas ejection nozzle in which said second solution tube is inserted.

11. A method for spraying and mixing a first solution and a second solution accelerating a coagulation reaction of the first solution, comprising:
ejecting said first solution from a first ejection port; and
ejecting said second solution from a second solution port, said second ejection port being disposed forward of said first ejection port with respect to a direction in which said first solution is ejected from said first ejection port.

12. The method of claim 11, further comprising
disposing a first solution tube terminating at said first ejection port in a first gas ejection nozzle, said first gas ejection nozzle being oriented in said gas ejecting direction;
disposing a second solution tube terminating at said second ejection port in a second gas ejection nozzle, said second gas ejection nozzle being oriented in a direction which is the same or substantially the same as said gas ejecting direction; and
spraying said first and second solutions ejected from said first and second ejection ports, respectively.

13. The method of claim 12, wherein
each of said gas ejection nozzles is defined by a cylindrical wall extending in a direction which is the same or substantially the same as said ejecting direction;
at least of a peripheral surface of a distal end of said gas ejection nozzle in which said first solution tube is inserted is spaced apart from that of said gas ejection nozzle in which said second solution tube is inserted.
